# EUROPEAN PATENT APPLICATION

(11) **EP 2 832 221 A1**
(43) Date of publication of application: **04.02.2015**
(21) Application number: 12872831.8
(22) Date of filing: 27.03.2012
(51) Int. Cl.: A01K 67/027, C12N 15/09

(54) **HUMANIZED MOUSE**

(71) Applicant: Trans Genic Inc., Chuo-ku, Kumamoto-shi, Kumamoto 862-0976 (JP); National University Corporation Kumamoto University, Kumamoto-shi, Kumamoto 860-8555 (JP)
(72) Inventor: YAMAMURA, Kenichi, Kumamoto-shi Kumamoto 860-0811 (JP); ARAKI, Kimi, Kumamoto-shi Kumamoto 860-0811 (JP); OKADA, Seiji, Kumamoto-shi Kumamoto 860-0811 (JP); SHIMONO, Akihiko, Kobe-shi Hyogo 650-0047 (JP)
(74) Representative: Woods, Geoffrey Corlett
(86) International application number: PCT/JP2012/058790
(87) International publication number: WO 2013/145331

(57) **Abstract**

The present invention provides embryonic stem cells obtainable from an embryo of an immunodeficient mouse which is deficient in both Rag2 and Jak3 genes by culture in the presence of a GSK3 inhibitor and an MEK inhibitor, as well as a transgenic mouse, which is created with the use of these embryonic stem cells.

## Description

### TECHNICAL FIELD

The present invention relates to embryonic stem cells (ES cells) taken from an immunodeficient mouse, and a mouse with a humanized liver.

### BACKGROUND ART

The liver is an organ which plays a dominant role *in vivo,* e.g., in metabolism, excretion, detoxication, and maintenance of body fluid homeostasis. The liver is the only regenerating organ in the body and is known to have the regeneration ability to recover its initial weight even if about 80% of its total weight is excised.

The liver has a wide range of functions and hence many genes are expressed in the liver, so that there are also many hereditary diseases caused by abnormalities in the genes expressed in the liver.

In some cases where liver functions become abnormal due to liver diseases and others, there is no effective therapy except for liver transplantation. For this reason, there has been an increasing necessity to predict human blood metabolites at the early stage of onset. To properly predict blood metabolites and liver functions, there is a need for the development of an animal with a humanized liver.

Among previous reports on the preparation of human liver model mice, for example, Heckel et al. have reported transgenic mice (Tg(Alb-Plau)) carrying a construct (Alb-Plau) composed of the urokinase-type plasminogen activator (Plau) gene linked to the albumin (Alb) promoter (Non-patent Document 1: Heckel et al. Cell 62:447-456, 1990)). However, these mice cannot be used for experiments because they will die within 4 days after birth due to hemorrhage in their intestinal tract and elsewhere. On the other hand, the same research group has succeed in establishing lines of survivors among Tg(Alb-Plau) mice and has reported a case where the liver was regenerated from liver cells which were deficient in the Alb-Plau gene during liver cell division (Non-patent Document 2: Sandgren et al. Cell 66:245-256, 1991). Moreover, there is a report showing successful transplantation of Tg(Alb-Plau) with mature liver cells from a transgenic mouse (Tg(MT-nLacZ) mouse) carrying a construct composed of the lacZ gene linked to the metallothionein promoter, i.e., a mouse whose liver cells serving as a donor were labeled with the marker gene lacZ (Non-patent Document 3: Rhim et al. Science 263:1149-1152, 1994).

In addition, there are reports on the transplantation of immunodeficient mice with human liver cells, as exemplified by a report in which Rag2-/- gene-deficient immunodeficient mice were transplanted with liver cells, followed by infection experiment with hepatitis B virus (HBV) (Non-patent Document 4: Dandri et al. Hepatology 33:981-988, 2001), or a report in which Tg(Alb-Plau) mice were crossed with SCID mice, which are immunodeficient mice, and the resulting immunodeficient SCID mice (Tg(Alb-Plau)) were then transplanted with human liver cells (Tg(Alb-Plau);SCID)), followed by infection experiment with hepatitis C virus (Non-patent Document 5: Mercer et al. Nature Med. 7:927-933, 2001).

Further, Tateno et al. have reported that albumin enhancer/promoter urokinase plasminogen activator transgenic mice (uPA mice) undergoing liver failure were crossed with SCID mice to prepare uPA/SCID transgenic mice homozygouse for both characters (Non-patent Document 6: Tateno et al. Amer. J. Pathol 165:901-912, 2004). This report discusses improved techniques for transplantation of human liver cells into Tg(Alb-Plau;SCID), in which Futhan treatment is used to eliminate the effects of complements derived from human liver cells to thereby reduce the mortality even at high chimerism.

Moreover, there is a report on the study which demonstrates the possibility of Rag2 gene-deficient immunodeficient mice as a model for gene therapy (Non-patent Document 7: Orthopedic Surgery and Traumatology "Series IV of Orthopedic Diseases from the Molecular Level, Somatic Cell Cloning Technology and Regenerative Medicine" Vol. 45, NO. 11, PAGE. 1040-1041,2002).

However, these model mice do not serve as a liver cell model in which 100% of the cells have been replaced with cells of human origin, because host mouse liver cells are left therein. In addition, cells of human origin do not always regenerate, so that cells of human origin should be transplanted. Moreover, when liver cells of mouse origin are left, human liver functions cannot be verified sufficiently.

On the other hand, for establishment of NOG mouse-derived ES cell lines for germ-line transmission, some attempts have also been made to establish ES cells by using differentiation signal inhibitors (PD0325901, CHIR99021) (Non-patent Document 8: Abstracts of the Annual Meeting of the Japanese Association for Laboratory Animal Science, Vol. 58th, Page 210, 2011).

However, NOG mice are difficult to obtain in large number for use in experiments because they are difficult to breed.

### Prior Art Documents

### Non-patent Documents

Non-patent Document 1: Heckel et al. Cell 62:447-456, 1990
Non-patent Document 2: Sandgren et al. Cell 66:245-256, 1991
Non-patent Document 3: Rhim et al. Science 263:1149-1152, 1994
Non-patent Document 4: Dandri et al. Hepatology 33:981-988, 2001
Non-patent Document 5: Mercer et al. Nature Med. 7:927-933, 2001
Non-patent Document 6: Tateno et al. Amer. J. Pathol 165:901-912, 2004
Non-patent Document 7: Orthopedic Surgery and Traumatology "Series IV of Orthopedic Diseases from the Molecular Level, Somatic Cell Cloning Technology and Regenerative Medicine" Vol. 45, NO. 11, PAGE. 1040-1041,2002 (in Japanese)
Non-patent Document 8: Abstracts of the Annual Meeting of the Japanese Association for Laboratory Animal Science, Vol. 58th, Page 210, 2011 (in Japanese)

### SUMMARY OF THE INVENTION

### PROBLEM TO BE SOLVED BY THE INVENTION

The object of the present invention is to provide embryonic stem cells (ES cells) taken from an immunodeficient mouse, and a mouse with a humanized liver.

### MEANS TO SOLVE THE PROBLEM

As a result of extensive and intensive efforts made to solve the problems stated above, the inventors of the present invention have found that embryonic stem cells which can be used to create a mouse most suitable for human liver cell transplantation are obtained from an embryo of an immunodeficient mouse which is deficient in both Rag2 and Jak3 genes. This finding led to the completion of the present invention.

Namely, the present invention is as follows.
(1) An embryonic stem cell obtainable from an embryo of an immunodeficient mouse which is deficient in both Rag2 and Jak3 genes by culture in the presence of a GSK3 inhibitor and an MEK inhibitor.
(2) The embryonic stem cell according to (1) above, which is deposited under Accession No. NITE BP-1297.
(3) The embryonic stem cell according to (1) or (2) above, which is engineered to have the estrogen receptor gene and the diphtheria toxin gene.
(4) The embryonic stem cell according to (3) above, wherein the endogenous growth hormone gene in the cell is replaced with that of human origin.
(5) The embryonic stem cell according to (4) above, wherein an endogenous drug-metabolizing enzyme gene in the cell is further replaced with that of human origin.
(6) The embryonic stem cell according to (5) above, wherein the endogenous drug-metabolizing enzyme gene in the cell is at least one selected from the group consisting of Cyp3a11, Cyp3a13, Cyp3a25 and Cyp3a41.
(7) A mouse, which is created with the use of the embryonic stem cell according to (1) or (2) above.
(8) A transgenic mouse, which is created with the use of the embryonic stem cell according to any one of (3) to (6) above.
(9) The mouse according to (8) above, which develops liver cell injury upon administration of an antiestrogen.
(10) A mouse with a humanized liver, wherein the mouse according to (7) above is transplanted with liver cells of human origin and also administered with an antiestrogen to eliminate liver cells originating from the mouse.
(11) The mouse according to (9) above, wherein the liver cells of human origin are derived from a patient with a liver disease.
(12) A human liver disease model mouse, which consists of the mouse according to (10) above.
(13) A method for preparing an immunodeficient mouse-derived embryonic stem cell, which comprises culturing an embryo of an immunodeficient mouse which is deficient in both Rag2 and Jak3 genes in the presence of a GSK3 inhibitor and an MEK inhibitor.
(14) A method for creating a liver injury model mouse, which comprises administering an antiestrogen to the mouse according to (8) above.
(15) A method for creating a mouse with a humanized liver, which comprises transplanting liver cells of human origin into the mouse according to (8) above and also administering an antiestrogen to eliminate liver cells originating from the mouse.
(16) The method according to (15) above, wherein the liver cells of human origin are derived from a patient with a liver disease.

### EFFECTS OF THE INVENTION

The present invention provides embryonic stem cells for establishment of a mouse most suitable for human liver cell transplantation. The embryonic stem cells of the present invention can be engineered to have various human genes related to liver functions to thereby establish a humanized liver model mouse. Thus, a mouse established from the embryonic stem cells of the present invention is very useful in that it can be used for human liver cell transplantation and achieves 100% humanization.

### BRIEF DESCRIPTION OF THE DRAWINGS

Figure 1 shows lox mutants.
Figure 2 shows the Dre/rox system.
Figure 3 shows a scheme for construction of a replacement vector used for introduction of the human growth hormone gene into ES cells.
Figure 4 shows a scheme for construction of a replacement vector used for introduction of a human drug-metabolizing enzyme gene into ES cells.
Figure 5 shows a scheme for the process starting from introduction of the diphtheria toxin gene into ES cells until cell death in mouse liver cells.
Figure 6 shows the site for transplantation of human liver cells into a mouse embryo.
Figure 7 shows mouse embryos transplanted with human liver cells.
   S1: Intraperitoneal administration of an anesthetic agent, S2: Fetus exposed extraperitoneally by laparotomy
   A: Yolk sac vessel into which cells are to be injected, B: Cell injection site (yellow arrows), C: Liver after blue dye injection (seen in blue), D: Excised livers (left: liver after blue dye injection, right: liver without dye injection)

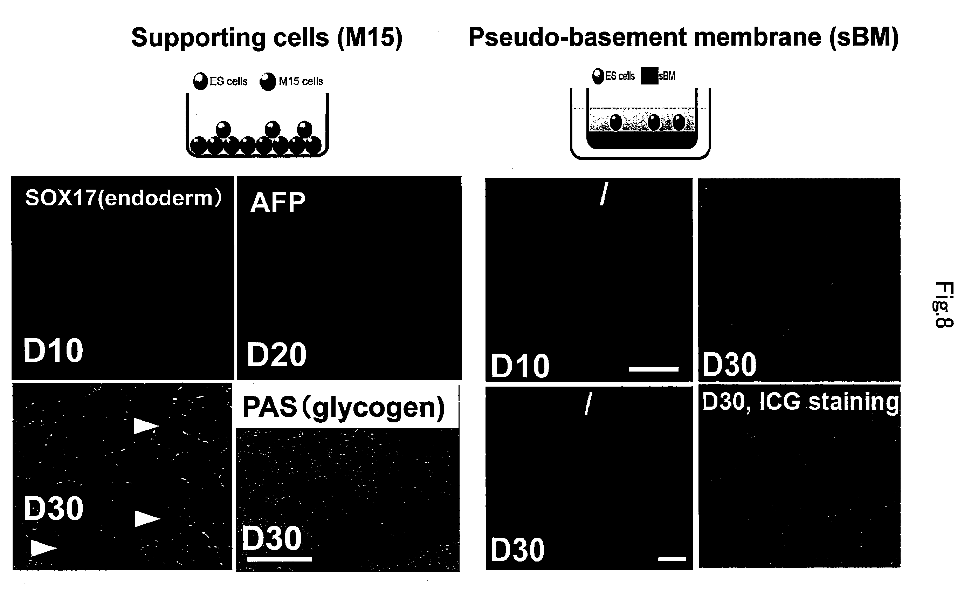

Figure 8 shows liver cells induced to differentiate from iPS cells.

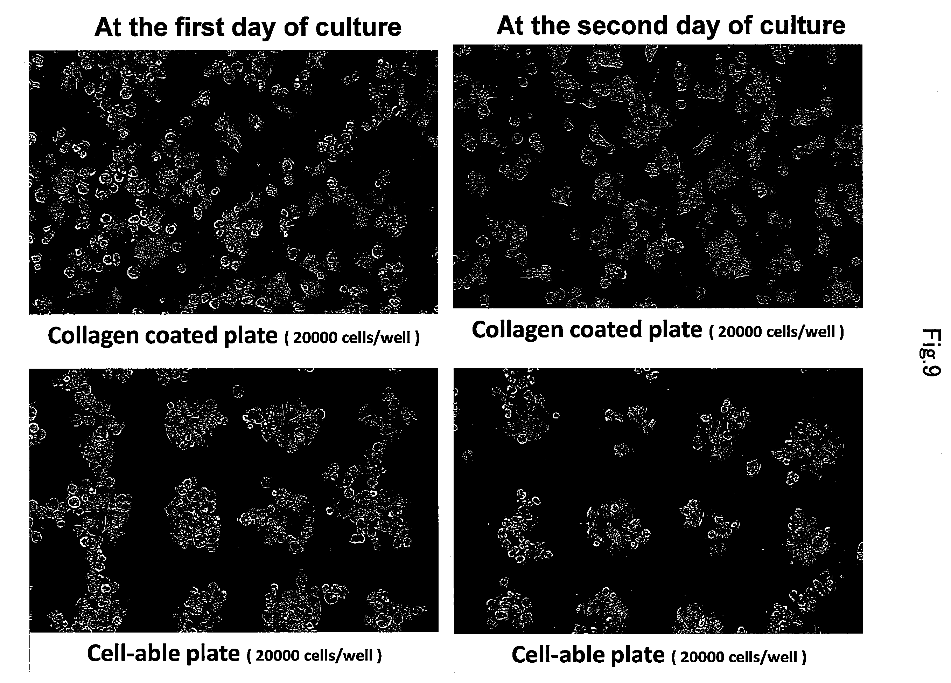

Figure 9 shows liver cells induced to differentiate from iPS cells.

### DESCRIPTION OF EMBODIMENTS

The present invention will be described in more detail below.

### 1. Summary

The present invention has been made to provide embryonic stem cells established from an immunodeficient mouse which is deficient in both Rag2 and Jak3 genes, and to establish a mouse with a humanized liver from these embryonic stem cells.

In the present invention, an embryo taken from the immunodeficient mouse is cultured in the presence of a GSK3 inhibitor and an MEK inhibitor to thereby successfully establish ES cells.

A BALB/c mouse deficient in Rag2 and Jak3 (BALB/c;Rag2-/-;Jak3-/-: hereinafter referred to as "BRJ mouse") is an immunodeficient mouse which lacks T cells, B cells, NK cells and NKT cells and has the genetic background of BALB/c mice. When this mouse is transplanted with human cells, these cells are engrafted in the mouse body, so that the resulting mouse is humanized at the cellular level.

However, in such a humanized mouse, cells originating from the host mouse are left therein, and hence all of its organs are not replaced with those of human origin. For this reason, such a humanized mouse is not necessarily optimized for functional analysis or study on these organs. Moreover, various genetic modifications are required to prepare an optimized mouse, although a whole mouse cannot be used for this purpose.

Thus, for establishment of a mouse with a liver whose cells have all been humanized, the present invention aims to establish a genetically modified mouse which is most suitable for humanization. As a result of extensive and intensive efforts aimed at humanization from the early stage of ontogeny in this genetically modified mouse, the inventors of the present invention have succeeded in establishing embryonic stem cells (hereinafter referred to as "ES cells") from BRJ mice. The inventors of the present invention have also succeeded in preparing a chimeric mouse using the ES cells to thereby prepare a germ-line chimeric mouse for germ-line transmission. Next, for maintenance of liver functions over a long period of time and for confirmation of the safety, the present invention aims to establish a mouse with a human normal liver. Moreover, for establishment of a disease model having the same symptoms as seen in human patients with liver disease and for analysis of the pathology, the present invention aims to establish a mouse with a human mutated liver. Furthermore, for development of a novel therapy used for a wide range of purposes, the present invention aims to establish a model mouse optimized for human diseases.

### 2. Preparation of BRJ mouse

The immunodeficient mouse to be used is a mouse whose Rag2 and Jak3 genes are both knocked out, which has already been established (Ono A, Hattori S, Kariya R, Iwanaga S, Taura M, Harada H, Suzu S, Okada S. Comparative study of human hematopoietic cell engraftment into BALB/c and C57BL/6 strain of rag-2/jak3 double-deficient mice. J Biomed Biotechnol 2011:539748, 2011).

This mouse can be obtained by crossing between a Rag2 gene-deficient mouse and a Jak3 gene-deficient mouse.

The Rag (recombination activating gene) 2 gene is expressed in immature lymphocytes and has functions essential for rearrangement of immunoglobulin genes and T cell receptors, and is therefore a gene indispensable for maturation of T cells and B cells.

How to prepare a mouse knockout of this Rag2 gene and details on this gene can be found in, e.g., Shinkai Y. et al., Cell. 1992 Mar 6;68(5):855-67, Chen J. et al., Curr Opin Immunol. 1994 Apr;6(2):313-9. In general, such a mouse can be prepared by any technique well known in the art, e.g., the technique using a targeting vector (Capecchi, M. R., Science, (1989) 244, 1288-1292). This technique is based on homologous recombination between the Rag2 gene in mouse ES cells and a gene on the targeting vector.

Jak3 (Janus kinase 3), which is a non-receptor tyrosine kinase, is a protein having the function of associating with the intracellular region of the common γ chain, which is common to IL-2, IL-4, IL-7, IL-9, IL-15 and IL-21 receptors, to thereby transduce signals into cells through the common γ chain. Signals through the common γ chain and Jak3 are essential for NK cells, and hence damage to this pathway will cause NK cell deficiency. Namely, when the Jak3 gene is knocked out, NK activity can be eliminated.

Details on the Jak3 gene and the common γ chain gene can be found in, e.g., Park SY. et al., Immunity. 1995 Dec;3(6):771-82, Suzuki K. et al., Int Immunol. 2000 Feb;12(2):123-32, and it is possible to obtain a mouse which is deficient in the Jak3 gene and loses NK activity, by reference to these documents.

It should be noted that a Rag2-deficient (-/-) mouse and a Jak3-deficient (-/-) mouse are also available from the Institute of Resource Development and Analysis, Kumamoto University, Japan. These mice can be back-crossed with commercially available BALB/c mice to thereby obtain a BALB/c Rag2-deficient (-/-) mouse and a BALB/c Jak3-deficient (-/-) mouse, respectively, each having the same genetic background as BALB/c mice.

To prepare a double knockout mouse deficient in both Rag2 and Jak3 genes, the BALB/c Rag2-deficient mouse and the BALB/c Jak3-deficient mouse are first crossed with each other to obtain F1 mice, followed by crossing between F1 mice to obtain F2 mice. From among these mice, a double-deficient, i.e., Rag2-deficient (-/-) and Jak3-deficient (-/-) mouse (BRJ mouse) may then be selected. As to techniques for BRJ mouse selection, for example, deficiencies in both Rag2 and Jak3 genes can be confirmed by PCR or Southern blotting.

### 3. Establishment of ES cells

The ES cells of the present invention can be obtained from embryos taken from BRJ mice obtained as above by culture in the presence of a GSK3 inhibitor and an MEK inhibitor.

First, from female BRJ mice after fertilization, fertilized eggs or two-cell embryos are obtained by culture or blastocysts are obtained directly. Fertilization may be accomplished by natural crossing or *in vitro* fertilization techniques. In the case of *in vitro* fertilization, ova obtained by superovulation of female mice and sperm taken from male mice may be cultured together.

Then, the collected blastocysts or inner cell mass may be cultured in a medium for animal cell culture in the presence of a GSK-3 inhibitor and an MEK inhibitor for about 1 to 3 weeks, preferably 14 to 18 days.

GSK-3 (glycogen synthase kinase 3), which is a serine/threonine protein kinase, is an enzyme acting on many signaling pathways responsible for glycogen production, apoptosis, stem cell maintenance and other events. Examples of a GSK-3 inhibitor include CHIR99021 (available from Wako Pure Chemical Industries, Ltd., Japan), 6-bromoindirubin-3'-oxime (BIO) (available from Wako Pure Chemical Industries, Ltd., Japan) and so on. Such a GSK-3 inhibitor may be added to the medium in an amount of 0.1 to 10 µM (micromolar), preferably 0.3 to 3 µM. The timing of GSK-3 inhibitor addition to the medium is not limited in any way, but it is preferably added from the beginning of blastocyst culture.

An MEK inhibitor is a protein kinase inhibitor which inhibits MAP Kinase Kinase (MEK) activity and suppresses ERK1/ERK2 activation. Examples of an MEK inhibitor include PD0325901 (available from Wako Pure Chemical Industries, Ltd., Japan), U0126 (available from Promega) and so on. The PD0325901 inhibitor may be added to the medium in any amount, for example, 3 µM.

Culture may be accomplished under any conditions, for example, at 37°C in a 5% CO₂ atmosphere. Subculture may be conducted at an interval of 3 to 4 days on mouse embryo fibroblast (MEF) feeders or on collagenase I-coated plates.

Examples of the above medium include GMEM medium (Glasgow's Minimal Essential Medium), DMEM (Dulbecco's Modified Eagle's Medium), RPMI 1640 medium and so on. The culture medium may be supplemented as appropriate with an additional ingredient(s) selected from KSR (knockout serum replacement), fetal bovine serum (FBS), basic fibroblast growth factor (bFGF), β-mercaptoethanol, nonessential amino acids, glutamic acid, sodium pyruvate and antibiotics (e.g., penicillin, streptomycin), etc.

Culture may be continued for a given period of time, followed by incubation in a medium containing EDTA or collagenase IV to collect ES cells. The collected ES cells may optionally be subcultured several times by culture in the presence or absence of feeder cells. It should be noted that inner cell mass culture under feeder-free conditions may be conducted in an MEF-conditioned medium.

The cultured ES cells may usually be identified using their marker genes. Examples of marker genes in ES cells include Oct3/4, alkaline phosphatase, Sox2, Nanog, GDF3, REX1, FGF4 and so on. The presence of marker genes or gene products may be detected by any technique such as PCR or Western blotting.

Moreover, to determine whether or not the ES cells of the present invention are obtained as desired, whether they are of BALB/c origin can be confirmed by SNP marker detection, while whether they are Rag2-deficient and Jak3-deficient can be confirmed by PCR or Southern blotting analysis. For example, a database of mouse SNPs is published at http://www.broadinstitute.org/snp/mouse, and when SNP information is compared against this database, the ES cells can be confirmed to be of BALB/c origin, while if the ES cells are found to be deficient in Rag2 and Jak3 genes, they are determined to be the ES cells of the present invention.

The thus obtained ES cells were designated as "BRJ8" and internationally deposited under the Budapest Treaty on March 23, 2012 (receipt date) with the National Institute of Technology and Evaluation, Patent Microorganisms Depositary (Patent Microorganisms Depositary, Department of Biotechnology of NITE, 2-5-8 Kazusakamatari, Kisarazu-shi, Chiba 292-0818, Japan). Their Accession No. is "NITE BP-1297."

Detailed information on the above ES cells is as follows.

Articles have already been published about how to establish ES cells with a GSK inhibitor and an MEK inhibitor, and the resulting ES cells inherit genetic characters of their original lines and contain their respective unique features. Although there are various lines of immunodeficient mice, they have mutually different gene mutations in addition to their original genetic background, so that different lines have different inherent features. For example, NOG mice, for which reports have been issued, are mice of NOD strain with SCID and a deficiency in the IL2 receptor common gamma (IL2R-γ) gene. NOD mice originally lack complements. A responsible gene for SCID is Prkdc (DNA-dependent protein kinase, catalytic subunit), and this gene is necessary for rearrangement of immunoglobulin genes and T cell receptors. Thus, a mutation in this gene will inhibit the formation of B lymphocytes and T lymphocytes. Moreover, IL2R-γ is a common molecule that constitutes receptors for interleukins such as IL-2, IL-4, IL-7, IL-9, IL-15 and IL-21. Thus, a deficiency in this molecule will inhibit the transduction of signals mediated by these interleukins, so that immune responses cannot be induced. Taken together, not only lack of complements, B lymphocytes and T lymphocytes, but also reduced functions of macrophages and/or dendritic cells are observed, thus resulting in a more severe immunodeficient state. However, due to severe immunodeficiency, even opportunistic infection pathogens, which do not cause any problem at all in normal mice, will be responsible for death in some cases, or thymoma will occur at high rate.

On the other hand, BRJ mice are mice of BALB/c strain with deficiencies in the Rag2 and Jak3 genes, and are known for high engraftment rate of transplanted cells. Rag2 is a gene necessary for rearrangement of immunoglobulin genes and T cell receptors, as in the case of Prkdc, while Jak3 is a gene located downstream of IL2R-γ, so that deficiencies in these genes result in a severe immunodeficient state. However, BRJ mice are relatively easy to keep and breed.

In preliminary experiments, attempts were made to establish ES cells in the conventionally used GMEM-KSR medium, but only a strain showing very poor growth could be established. Even when used to prepare a chimeric mouse, this strain resulted in chimerism as low as 50% and did not contribute to the germ line. In contrast, in the present invention, a GSK3 inhibitor and an MEK inhibitor, which are considered to be effective for maintenance of the undifferentiated state of ES cells, were added to the medium to thereby achieve the establishment of the desired ES cells. The ES cells of the present invention are high in viability and also high in chimerism. This is because the ES cells of the present invention successfully maintain their undifferentiated state in comparison with ES cells prepared by conventional techniques. One of the important signals responsible for differentiation of ES cells is the ERK/MEK pathway from FGF4 through FGF receptors. Namely, ERK acts as a differentiation signal. On the other hand, GSK-3 stimulates Wnt signals through phosphorylation of β-catenin to thereby induce differentiation. Thus, by using two inhibitors (2i), i.e., a strong MEK inhibitor (PD0325901) and a GSK3 inhibitor, the ES cells of the present invention can be prevented from differentiation and hence maintain their pluripotency.

### 4. Genetic modifications

To establish a genetically modified mouse which is most suitable for humanization, endogenous genes should be replaced with those of human origin at the stage of ES cells, but not in adult mice, or ES cells should be transformed with human genes, followed by creation of a mouse from the thus genetically modified and/or transformed ES cells.

Thus, in the present invention, for transformation of ES cells with desired genes or for replacement of endogenous genes in ES cells with human genes, homologous recombination with the following systems may be used: the bacteriophage-derived recombination system Cre-loxP, the Vibrio sp.-derived recombination system VCre-Vlox, the Cre homolog-mediated recombination system Dre/rox, or any system modified from these recombination systems.

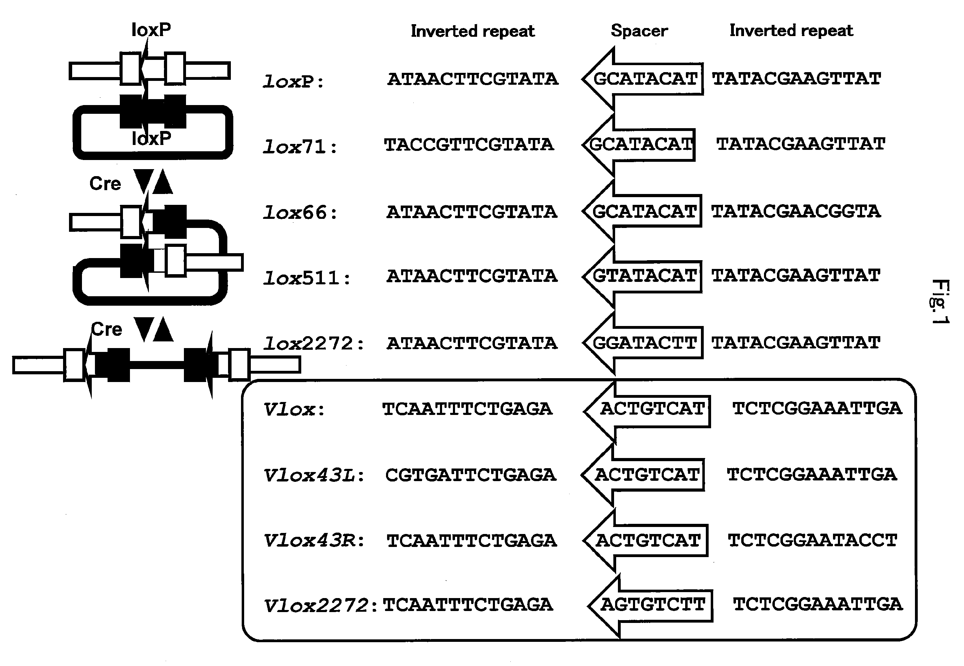

loxP (locus of crossing (X-ring) over, P1) is a sequence of 34 nucleotides (5'-ATAACTTCGTATA GCATACAT TATACGAAGTTAT-3') (SEQ ID NO: 1), in which sequences of the 5'-terminal 13 nucleotides (referred to as inverted repeat 1) and the 3'-terminal 13 nucleotides (referred to as inverted repeat 2) each constitute an inverted repeat, and a sequence represented by "GCATACAT" which is called a 8-nucleotide spacer is sandwiched between the above inverted repeats 1 and 2 (Figure 1). The term "inverted repeat" is intended to mean a sequence, one of whose terminal segments is complementary to the other terminal segment in the direction opposite to each other, with sandwiching a spacer which serves as their boundary.

Cre (causes recombination) is intended to mean a recombination enzyme (also referred to as a recombinase) which causes gene recombination, and it recognizes the above repeats to cleave the spacer in such a cleavage fashion that "cataca" in the spacer segment is left as a cohesive end.

On the other hand, in the case of bacteria, recombination will occur between their two loxP sites to cause insertion or deletion reaction (Figure 1). If insertion reaction can be caused in mammalian cells, any gene can be inserted subsequently, thus resulting in a significantly wider range of applications. Since mammalian cells have large nuclei, circular DNA whose loxP has been deleted will diffuse and little insertion reaction is observed.

For this reason, the inventors of the present invention have attempted to introduce a mutation into a loxP sequence to cause insertion reaction such that once a gene has been inserted into the genome, the inserted gene cannot be deleted (i.e., cannot be eliminated from the genome), and have designed several types of loxP mutants (lox66, lox71, lox511, lox2272) for this purpose (Figure 1). These loxP mutants are known (WO01/005987, JP 2007-100 A).

Moreover, in the present invention, systems under the name Vlox can also be used. Vlox refers to a Vibrio sp.-derived recombination system, VCre-Vlox (Suzuki, E., Nakayama, M. VCre/VloxP and SCre/CloxP: new site-specific recombination systems for genome engineering. Nucleic Acid Res. 2011, 1-11), and Vlox43L, Vlox43R, Vlox2272 and so on are available for use (Figure 1).

The nucleotide sequences of loxP and loxP mutants as well as Vlox systems are shown below (Figure 1).
loxP: ATAACTTCGTATAGCATACATTATACGAAGTTAT (SEQ ID NO: 1)
lox71: TACCGTTCGTATAGCATACATTATACGAAGTTAT (SEQ ID NO: 2)
lox66: ATAACTTCGTATAGCATACATTATACGAACGGTA (SEQ ID NO: 3)
lox511: ATAACTTCGTATAGTATACATTATACGAAGTTAT (SEQ ID NO: 4)
lox2272: ATAACTTCGTATAGGATACTTTATACGAAGTTAT (SEQ ID NO: 5)
Vlox: TCAATTTCTGAGAACTGTCATTCTCGGAAATTGA (SEQ ID NO: 6)
Vlox43L: CGTGATTCTGAGAACTGTCATTCTCGGAAATTGA (SEQ ID NO: 7)
Vlox43R: TCAATTTCTGAGAACTGTCATTCTCGGAATACCT (SEQ ID NO: 8)
Vlox2272: TCAATTTCTGAGAAGTGTCTTTCTCGGAAATTGA (SEQ ID NO: 9)

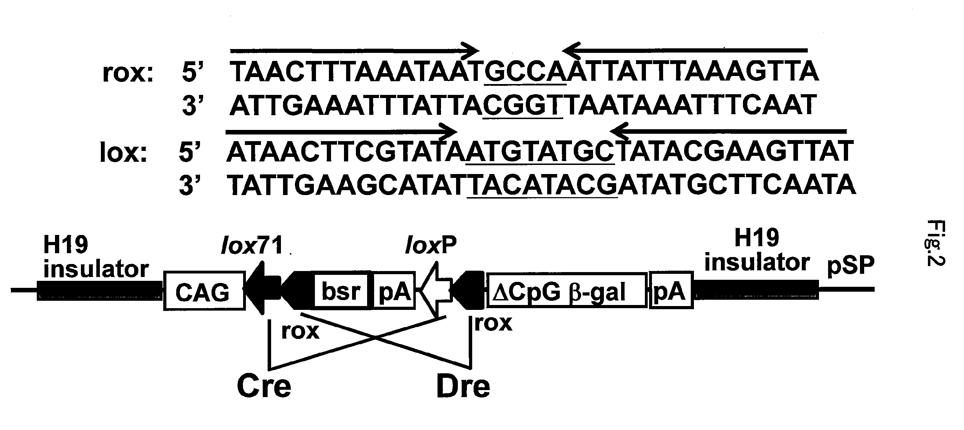

Further, in the present invention, the Dre/rox system can be used (Figure 2).

Dre refers to D6 site-specific DNA recombinase, which is an enzyme capable of recognizing the sequence of the rox site shown below (Sauer, B. and McDermott, Nucic Acid. Res. 32: 6086-6095, 2004). A recombination system based on this recombinase and the rox recognition sequence is referred to as the Dre/rox system. This system is closely related to the Cre-lox system although they differ in their DNA recognition specificity.

The nucleotide sequences of lox and rox are shown below (Figure 2).
rox: 5'-TAACTTTAAATAATGCCAATTATTTAAAGTTA-3' (SEQ ID NO: 10)
   3'-ATTGAAATTTATTACGGTTAATAAATTTCAAT-5' (SEQ ID NO: 11)
lox: 5'-ATAACTTCGTATAATGTATGCTATACGAAGTTAT-3' (SEQ ID NO: 12)
   3'-TATTGAAGCATATTACATACGATATGCTTCAATA-5' (SEQ ID NO: 13)

As described above, the present invention aims to establish a mouse with a human normal liver, and further aims to establish a liver disease model mouse. For this purpose, in the present invention, ES cells are genetically engineered to ensure that a toxin is expressed in the cytoplasm of mouse liver cells to induce cell death in the mouse liver cells. Moreover, for the reason that human liver cells should be transplanted and grown to create a mouse with a human normal liver, the mouse growth hormone gene in ES cells is replaced with the human growth hormone gene. In addition, for analysis of functions such as drug metabolism, mouse drug-metabolizing enzyme genes are replaced with human drug-metabolizing enzyme genes.

A mouse introduced with liver cell death loses liver functions. Thus, this mouse not only can be used as a liver injury model, but can also be used to obtain a mouse with a humanized liver upon transplantation of human normal liver cells.

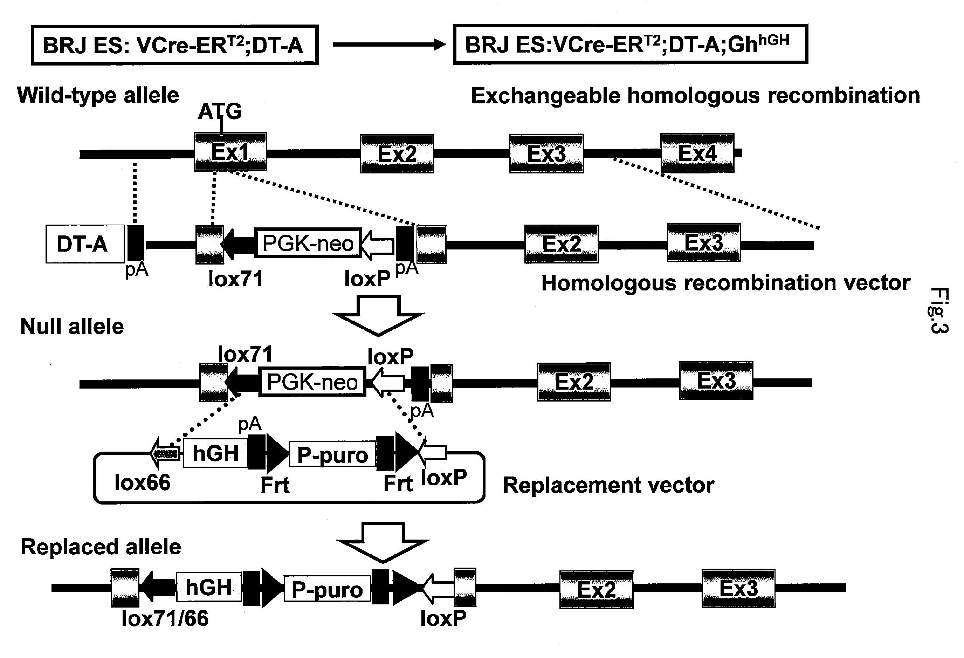

Figure 3 shows a scheme for construction of a homologous recombination vector for replacement of the mouse growth hormone (GH) gene with the human GH gene.

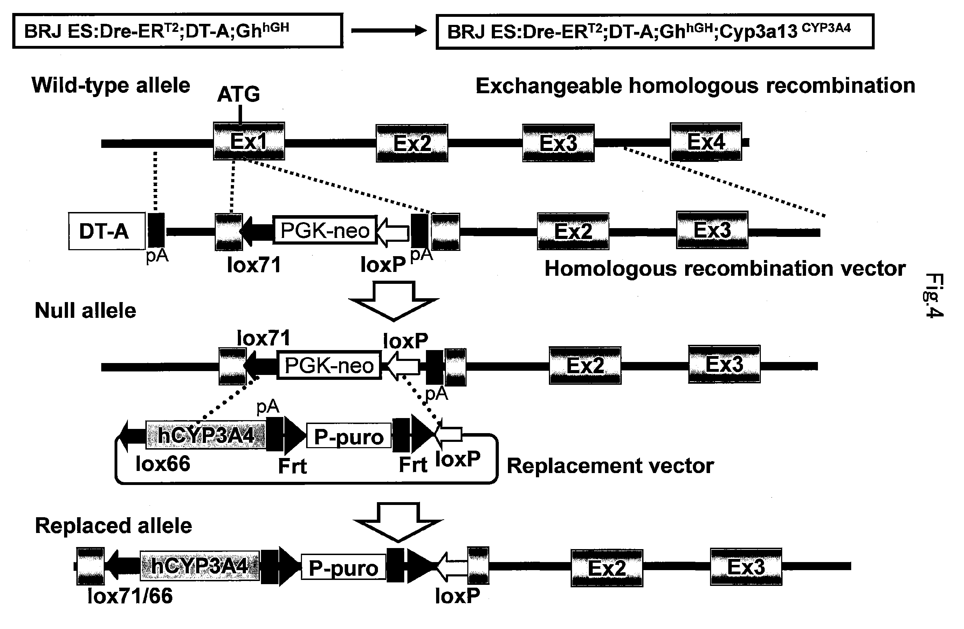

Likewise, Figure 4 shows a scheme for construction of a homologous recombination vector for replacement of the Cyp gene, a drug-metabolizing enzyme gene, with the human Cyp gene.

Replacement of mouse genes with the above human genes can be accomplished in accordance with the gene trapping method described in WO01/005987. For example, two-step gene trapping may be conducted using a vector prepared as described above.

The first step is a commonly used gene trapping method. In this commonly used gene trapping, the above trapping vector is introduced into ES cells to trap an endogenous gene inherently present in the ES cells. As a result, the endogenous gene in the ES cells is disrupted. Then, a human gene is ligated downstream of the lox sequence (e.g., lox66) on a plasmid (replacement vector), followed by the second step of gene trapping (Figures 3 and 4).

In the second step of gene trapping, the human gene (e.g., hGH, hCyp) ligated downstream of lox66 is introduced into the ES cells. As a result, the lox71 site in the trapping vector introduced during the first step causes recombination with lox66 in the vector introduced during the second step, whereby a modified gene containing a cassette composed of "(lox71/66)-(human gene)-(loxP)" can be introduced. It should be noted that the puromycin resistance gene (puro) may be ligated between the human gene and loxP.

According to this method, endogenous mouse genes can be replaced with human genes. Figures 3 and 4 show the replaced alleles.

In Figures 3 and 4, Ex1, Ex2, Ex3 and Ex4 represent exons 1 to 4, respectively, in the mouse growth hormone gene or the mouse Cyp3a13 gene, pA represents a polyA sequence, Frt represents a FLP recognition site, PGK-neo represents the neomycin resistance gene ligated with PGK promoter, and P-puro represents the puromycin resistance gene ligated with PGK promoter.

### 5. Preparation of chimeric mouse

Preparation of a chimeric mouse can be accomplished in a standard manner.

First, the above established ES cells or gene-introduced or -replaced ES cells are allowed to aggregate with an eight-cell embryo or injected into a blastocyst. The thus prepared embryo is referred to as a chimeric embryo, and this chimeric embryo is transplanted into the uterus of a pseudopregnant foster mother, which is then allowed to give birth, thereby preparing a chimeric mouse.

For example, to prepare a chimeric embryo, a female mouse treated with a hormone drug for superovulation may first be crossed with a male mouse. Then, after a given number of days have passed, an embryo at early development stage may be collected from the uterine tube or uterus. The collected embryo may be aggregated or injected with ES cells to prepare a chimeric embryo.

The term "embryo" as used herein is intended to mean an individual at any stage from fertilization to birth during ontogeny, including a two-cell embryo, a four-cell embryo, an eight-cell embryo, a morula stage embryo, a blastocyst and so on. An embryo at early development stage can be collected from the uterine tube or uterus at 2.5 days after fertilization for use as an eight-cell embryo and at 3.5 days after fertilization for use as a blastocyst.

For preparation of an aggregate using ES cells and an embryo, known techniques such as the microinjection method, the aggregation method and so on can be used. The term "aggregate" is intended to mean an aggregate formed from ES cells and an embryo gathering together in the same space, and includes both cases where ES cells are injected into an embryo and where an embryo is dissociated into separate cells and aggregated with ES cells.

In the case of using the microinjection method, the collected embryo may be injected with ES cells to prepare a cell aggregate. Alternatively, in the case of using the aggregation method, ES cells may be aggregated by being sprinkled over a normal embryo whose zona pellucida has been removed.

On the other hand, a pseudopregnant female mouse for use as a foster mother can be obtained from a female mouse with normal sexual cycle by crossing with a male mouse castrated by vasoligation or other techniques. The thus created pseudopregnant mouse may be transplanted in the uterine with a chimeric embryo prepared as described above and then allowed to give birth, thereby preparing a chimeric mouse.

From among the thus prepared chimeric mice, a male mouse derived from the ES cell-transplanted embryo is selected. After the selected male chimeric mouse has been matured, this mouse may be crossed with a pure-line female mouse. Then, if the coat color of the ES cell-derived mouse appears in the born pups, it can be confirmed that pluripotent stem cells have been introduced into the germ line of the chimeric mouse.

### 6. Preparation of humanized mouse

### (1) Preparation of genetically modified mouse which is most suitable for humanization

Such a transgenic mouse (i.e., genetically modified mouse) established by using gene-introduced or -replaced ES cells is a mouse serving as a base for establishment of a mouse with a 100% humanized liver, as described later.

To avoid rejection reactions, ES cells of NOJ mouse origin or ES cells of BRJ mouse origin are used.

### (i) NOJ (NOD/SCID/Jak3-/-) mouse: deficient in C3, T, B, NK and NKT

For NOJ mouse preparation, an NOD mouse is crossed with an SCID mouse to introduce SCID gene mutations into the genetic background of NOD, and further crossed with a Jak3-deficient mouse to obtain a mouse (NOJ mouse) having the genetic background of NOD with SCID and a deficiency in the Jak3 gene. This mouse is deficient in complement C3 and also deficient in T cells, B cells, NK cells and NKT cells.

### (ii) BRJ (BALB/c;Rag2-/-;Jak3-/-) mouse: deficient in T, B, NK and NKT

In the present invention, not only the above NOJ mouse, but also a BRJ mouse can be used. Such a BRJ mouse is a mouse having the genetic background of BALB/c mice introduced with deficiencies in the Rag2 and Jak3 genes. This mouse is deficient in T cells, B cells, NK cells and NKT cells. When compared with the NOJ mouse, the BRJ mouse is easy to breed, so that many mice can be produced.

### (2) Preparation of a liver injury model mouse

For preparation of a liver injury model mouse, an antiestrogen may be administered to cause toxin expression to thereby eliminate (kill) mouse liver cells, thus obtaining an injury model mouse losing its liver functions.

To kill mouse liver cells or to express Dre-ER^{T2} in the cytoplasm of mouse liver cells, the following constructs 1 and 2 are prepared. Dre-ER^{T2} is a vector carrying the Dre recombinase gene ligated to a mutated estrogen receptor gene modified to prevent binding with estrogen produced in the mammalian body.
Construct 1:
   CAG-ATG-rox-EGFP-rox-DT-A
Construct 2:
   SAP-Dre-ER^{T2}

Construct 1 is composed of (i) ATG, (ii) EGFP flanked by rox sites and (iii) DT-A (diphtheria toxin fragment A), which are ligated immediately downstream of the CAG promoter.

This construct is designed to ensure in-frame ligation between the initiation codon in EGFP and ATG located upstream of rox. This construct is also designed to remove the initiation codon in DT-A and to ensure in-frame ligation with ATG located upstream of rox.

Construct 2 is composed of Dre-ER^{T2} ligated immediately downstream of the promoter for liver cell-specific serum amyloid P component (SAP).

When these constructs 1 and 2 are co-introduced into the ES cells of the present invention, site-specific recombination will occur after tamoxifen administration, and diphtheria toxin will be expressed in a manner specific to liver cells, whereby cell death can be induced.

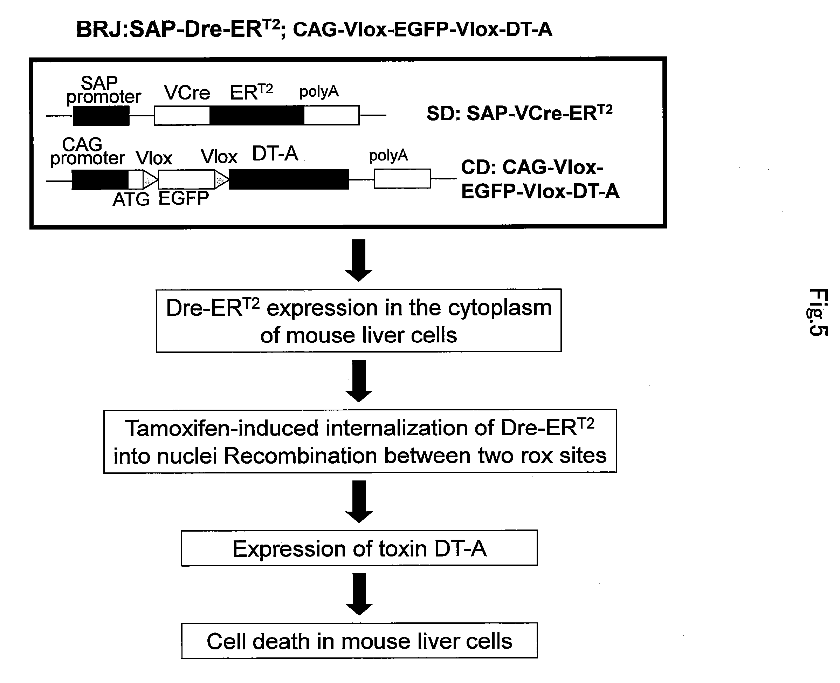

Namely, as a non-steroidal antiestrogen, for example, tamoxifen is a substance which has antitumor activity as a result of binding to the estrogen receptor in a manner competitive with estrogen to thereby exert an anti-estrogenic effect. When Dre-ER^{T2}-expressing humanized mice are administered with tamoxifen, Dre-ER^{T2} will be internalized into their nuclei by the action of tamoxifen. Recombination between two rox sites will occur to allow the promoter for the diphtheria toxin gene to function. As a result, toxin DT-A will be expressed to kill mouse liver cells (Figure 5).

Tamoxifen may be administered at any frequency and for any period as long as liver cells can be killed, although it is administered as follows, by way of example.

Tamoxifen is dissolved in ethanol and the resulting solution is diluted with sun flower oil (S5007, Sigma) to adjust the concentration at 7 mg/ml. This solution is used for administration to adults at a dose of 105 mg/kg body weight for successive 4 days via the intraperitoneal route.

### (3) Preparation of humanized mouse whose liver cells are replaced with human liver cells

For preparation of a mouse whose liver cells are replaced with human liver cells, mouse liver cells may be eliminated by antiestrogen administration and also human liver cells may be transplanted into a mouse, as described above, thus obtaining a humanized mouse whose liver cells are replaced with human liver cells.

Establishment of a mouse with a human normal liver is necessary to maintain liver functions over a long period of time and confirm the safety.

### (i) Preparation of ES cells in which the mouse growth hormone gene is replaced with the corresponding human gene

To ensure the growth of the transplanted human liver cells, the mouse growth hormone gene is replaced with the corresponding human gene at the stage of ES cells.

More specifically, gene replacement in ES cells may be accomplished in two steps, as described above.

In the first step, BRJ ES cells engineered to have SAP-Dre-ER^{T2} and CAG-rox-EGFP-rox-DT-A (hereinafter referred to as BRJ ES:SAP-Dre-ER^{T2};CAG-rox-EGFP-rox-DT-A) are used for homologous recombination to disrupt the mouse growth hormone gene at its initiation codon and also establish ES cells carrying lox71-PGK-neo-loxP integrated into this site (BRJ ES: :SAP-Dre-ER^{T2}; CAG-rox-EGFP-rox-DT-A; Gh^{neo}).

In the second step, these ES cells and a replacement vector may be used to establish ES cells carrying human growth hormone gene cDNA in place of the neo gene (BRJ ES:SAP-Dre-ER^{T2}; CAG-rox-EGFP-rox-DT-A; Gh^{hGH}).

The thus established ES cells may be used to obtain a mouse producing human growth hormone.

### (ii) Elimination of mouse liver cells and undifferentiated liver cells

The administration frequency and administration period of tamoxifen are the same as described above.

### (iii) Preparation of human liver cells to be transplanted

Human liver cells to be transplanted may be induced from iPS cells.

To obtain human liver cells, efficient techniques can be established for induction of endodermal and hepatic differentiation from human iPS cells with the use of supporting cells or an extracellular matrix.

iPS cells can be induced from somatic cells upon introduction of genes encoding 3 to 6 transcription factors (nucleus initialization factors) including members of Oct, Sox, Klf, Myc, Nanog, Lin and other families (Takahashi, K., et al. Induction of pluripotent stem cells from fibroblast cultures. Nat. Protoc. 2, 3081-9 (2007); Fusaki N, Ban H, Nishiyama A, Saeki K, Hasegawa M. Efficient induction of transgene-free human pluripotent stem cells using a vector based on Sendai virus, an RNA virus that does not integrate into the host genome. Proc Jpn Acad Ser B Phys Biol Sci. 2009; 85(8):348-62).

Members of the Oct family include, for example, Oct3/4, Oct1A, Oct6 and so on, with Oct3/4 being preferred.

Members of the Sox (SRY-related HMG box) family include, for example, Sox1, Sox2, Sox3, Sox7, Sox15 and so on, with Sox2 being preferred.

Members of the Klf (Kruppel-like factor) family include, for example, Klf1, Elf2, Klf4, Klf5 and so on, with Klf4 being preferred.

Members of the Myc family include c-Myc, N-Myc, L-Myc and so on, with c-Myc being preferred.

Nanog is a homeobox protein that is most highly expressed in the inner cell mass of blastocysts, but not expressed in differentiated cells.

Members of the Lin family include, for example, Lin28 which is used as a marker for undifferentiated human ES cells.

More specifically, preferred transcription factors are a combination of Oct3/4, Sox2, Klf4 and c-Myc (Takahashi, K. and Yamanaka, S., Cell 126, 663-676 (2006)), but it is also possible to use a combination of Oct3/4, Sox2 and Klf4 or a combination of Oct3/4, Sox2, Klf4 and L-Myc.

Examples of somatic cells include skin cells, liver cells, fibroblasts, lymphocytes and so on.

Techniques for gene transfer into somatic cells include, but are not limited to, lipofection, electroporation, microinjection, virus vector-mediated transfer, etc. Virus vectors used for this purpose include, for example, retrovirus vectors, lentivirus vectors, adenovirus vectors, adeno-associated virus vectors, Sendai virus and so on. It is also possible to use commercially available vectors, as exemplified by Sendai virus (DNAVEC).

In the case of using vectors, a gene to be introduced may also be operably linked to a regulatory sequence (e.g., a promoter, an enhancer) to ensure its expression. Examples of such a promoter include CMV promoter, RSV promoter, SV40 promoter and so on. These vectors may further comprise a positive selection marker such as a drug resistance gene (e.g., puromycin resistance gene, neomycin resistance gene, ampicillin resistance gene, hygromycin resistance gene), a negative selection marker (e.g., diphtheria toxin A fragment gene or thymidine kinase gene), IRES (internal ribosome entry site), a terminator, a replication origin and so on.

Somatic cells (e.g., 0.5 × 10⁴ to 5 × 10⁶ cells/100 mm dish) are transfected with a vector comprising the above nucleus initialization factors and cultured at about 37°C on MEF feeders or under feeder-free conditions, whereby iPS cells are induced after about 1 to 4 weeks.

Examples of a medium include GMEM medium (Glasgow's Minimal Essential Medium), DMEM (Dulbecco's Modified Eagle's Medium), RPMI 1640 medium, OPTI-MEMI medium and so on. The culture medium may be supplemented as appropriate with an additional ingredient(s) selected from KSR (knockout serum replacement), fetal bovine serum (FBS), activin-A, basic fibroblast growth factor (bFGF), retinoic acid, dexamethasone, β-mercaptoethanol, nonessential amino acids, glutamic acid, sodium pyruvate and antibiotics (e.g., penicillin, streptomycin), etc.

Culture may be continued for a given period of time, followed by incubation in a medium containing EDTA or collagenase IV to collect the cells, as in the case of ES cell culture. Under feeder-free conditions, the cells may be cultured on Matrigel-coated plates in an MEF-conditioned medium.

It is usual to induce differentiation from iPS cells into human liver cells via three steps. In principle, these three steps are as follows:
(a) induction from pluripotent stem cells into the endodermal lineage,
(b) induction from the endodermal lineage into immature liver cells, and
(c) induction from the immature liver cells into mature liver cells.

In the above step (a), activin A and Wnt signals appear to be important. Likewise, FGF and BMP appear to be important in the step (b), while hepatocyte growth factor, oncostatin and dexamethasone appear to be important in the step (c).

However, in the above steps (b) and (c), these important factors may be replaced as appropriate with DMSO or retinoic acid, and FGF4 or hydrocortisone and so on.

Transplantation of human liver cells may be conducted at 15.5 days of embryonic age or in adult mice at around 8 weeks after birth.

The number of human liver cells to be transplanted is preferably 10⁵ to 10⁶.

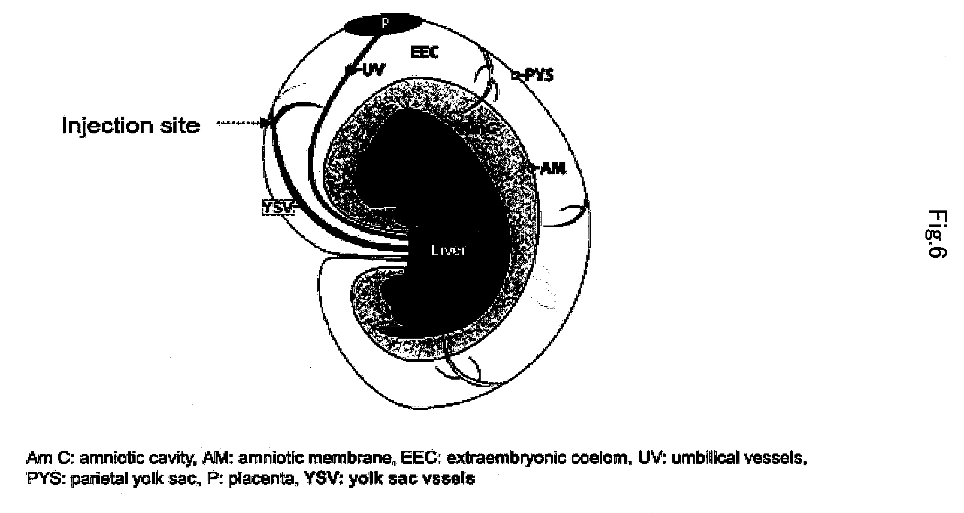

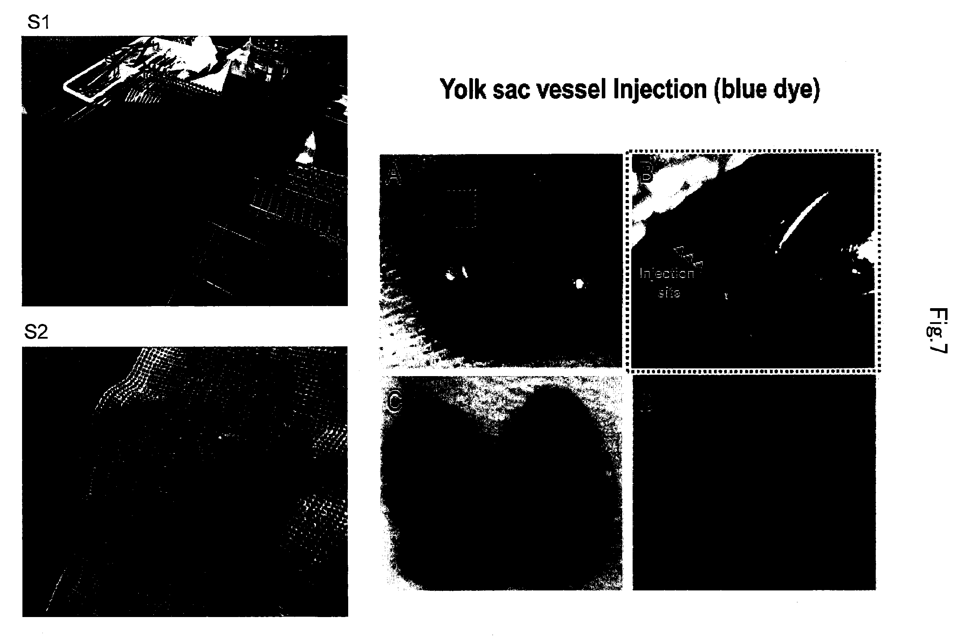

As to the route for transplantation of human liver cells, the cells may be transplanted through injection into the yolk sac vessel in the case of embryos (Figures 6 and 7). In the case of adult mice, the cells may be injected into the spleen.

### (iv) Growth of human liver cells

The mouse established using ES cells in which the mouse growth hormone gene has been replaced with the human growth hormone gene is able to produce human growth hormone. This human growth hormone acts on the transplanted human liver cells to promote their growth, whereby it is possible to establish a humanized liver mouse with a human liver of normal size.

To confirm that all (100%) of the mouse liver cells have been replaced with human liver cells, i.e., to confirm the absence of mouse liver cells, genes which are expressed in the mouse liver may be analyzed for their expression by RT-PCR or other techniques.

### (4) Evaluation of humanized liver mouse

To confirm that the liver has been humanized, the following characteristics may be tested either alone or in appropriate combination.

### (i) Verification of liver functions

Characteristics to be tested for verification of liver functions include, for example, those listed below. The test period is not limited in any way, but it is preferably one year or longer.
Proteins: total protein, ALB, TTT, ZTT, CRP, Haptoglobin, C3, C4
Non-protein nitrogen component: total bilirubin, direct bilirubin
Carbohydrate: glucose
Lipid: triglyceride, total cholesterol, HDL-cholesterol, LDL-cholesterol, ApoAI, ApoCII
Enzyme: lactate dehydrogenase (LDH), aspartate aminotransferase (AST (GOT)), alanine aminotransferase (ALT (GPT)), γ-glutamyltransferase (GGT), creatine kinase (CK), alkaline phosphatase (AP), amylase (AML)
Others: calcium, Fe, inorganic phosphate
ICG test: Indocyanine green (ICG) is intravenously administered and the ICG concentration in blood is measured over time to test the dye excretory function of the liver. ICG is bound to lipoproteins in blood and transported to the liver, and is taken up into liver cells during passing through sinusoids and then excreted into bile without being conjugated. Thus, the functions of the liver can be analyzed as a whole organ, but not as liver cells.
CT test: Morphological changes in the liver are tested.

### (ii) Drug metabolism

PCR array techniques are used to analyze the drug metabolism-related enzymes listed below.
Cytochrome P450: CYP11A1, CYP11B1, CYP11B2, CYP17A1, CYP19A1, CYP1A1, CYP1A2, CYP1B1, CYP21A2, CYP24A1, CYP26A1, CYP26B1, CYP26C1, CYP27A1, CYP27B1, CYP2A13, CYP2R1, CYP2S1, CYP2B6, CYP2C18, CYP2C19, CYP2C8, CYP2C9, CYP2D6, CYP2E1, CYP2F1, CYP2W1, CYP3A4, CYP3A43, CYP3A5, CYP3A7, CYP4A11, CYP4A22, CYP4B1, CYP4F11, CYP4F12, CYP4F2, CYP4F3, CYP4F8, CYP7A1, CYP7B1, CYP8B1.
Alcohol dehydrogenase: ADH1A, ADH1B, ADH1C, ADH4, ADH5, ADH6, ADH7, DHRS2, HSD17B10 (HADH2).
Esterase: AADAC, CEL, ESD, GZMA, GZMB, UCHL1, UCHL3.
Aldehyde dehydrogenase: ALDH1A1, ALDH1A2, ALDH1A3, ALDH1B1, ALDH2, ALDH3A1, ALDH3A2, ALDH3B1, ALDH3B2, ALDH4A1, ALDH5A1, ALDH6A1, ALDH7A1, ALDH8A1, ALDH9A1.
Flavin-containing monooxygenase: FMO1, FMO2, FMO3, FMO4, FMO5.
Monoamine oxygenase: MAOA, MAOB.
Prostaglandin-endoperoxide synthase: PTGS1, PTGS2.
Xanthine dehydrogenase: XDH.
Dihydropyrimidine dehydrogenase: DPYD.

### (iii) In vitro verification of liver cell functions

Since liver cells are of endodermal origin, test cells may be examined for time-dependent expression of genes which are expressed in the endodermal lineage and liver cells, accumulation of glycogen, expression of cytochrome enzymes and so on to thereby verify whether the test cells have human liver functions.

The time-dependent expression of genes which are expressed in the endodermal lineage and liver cells may be verified for Oct3/4, T, Gsc, Mixl1, Foxa2, Hex, Hnf4a, Hnf6, Afp, Alb, Ttr, αAT, etc. Techniques for their verification include, for example, commonly used Northern blotting, RT-PCR and Western blotting.

The secretory ability of liver cells may be verified by measuring ALB, transferrin, alpha1-antitrypsin and fibrinogen for their concentrations in the culture solution. Techniques for their verification include, for example, commonly used Western blotting or EIA (enzyme-immuno assay).

The accumulation of glycogen may be verified by PAS (periodic acid-Schiff) staining. Periodic acid selectively oxidizes glucose residues to generate aldehydes, causing a color change to red purple by the action of Schiff's reagent.

The expression of cytochrome enzymes may be verified by analysis of five major enzymes, i.e., CYP3A4, CYP1A2, CYP2C9, CYP2C19 and CYP2D6. Techniques for their verification include, for example, commonly used Northern blotting, RT-PCR and Western blotting.

### (5) Preparation of liver disease model mouse whose liver cells are replaced with human patient-derived liver cells

The mouse of the present invention may be transplanted with human patient-derived liver cells and also administered with an antiestrogen to eliminate liver cells originating from the mouse, whereby a human liver disease model mouse can be obtained.

Establishment of a mouse with a human mutated liver is necessary for establishment of a disease model having the same symptoms as seen in human patients and for pathology analysis. Moreover, a model optimized for human diseases is established and can be used for development of a novel therapy used for a wide range of purposes.

### EXAMPLES

The present invention will be further described in more detail by way of the following examples, although the present invention is not limited to these examples. It should be noted that all applications for induction of liver cells from iPS cells, establishment of iPS cells from patients with human familial amyloid polyneuropathy or patients with human propionic acidemia, and transplantation experiments of the induced human liver cells into mice were approved by the ethical committee, the animal research committee, and the safety committee on recombinant DNA experiments of class 2.

### [Example 1]

### Establishment of ES cells

In this example, for establishment of a humanized optimal mouse most suitable for human liver cell transplantation, ES cell lines were established from BRJ mouse embryos, and mouse strains thereof were also established.

### (1) Establishment of BALB/c;Rag2-/-;Jak3-/- (BRJ) mice and ES cell lines thereof

A Rag2-deficient mouse and a Jak3-deficient mouse were crossed with each other to establish a double-deficient mouse BRJ (Ono A, et al. J Biomed Biotechnol 2011; 539748, 2011. doi: 10.1155/2011/5397481)). The established BRJ mice were used for *in vitro* fertilization to obtain 64 blastocyst embryos, which were then cultured in the conventionally used GMEM-KSR medium (14% KSR, 1% FBS, 1000 U/ml LIF in GMEM) in an attempt to establish cell lines, but only two lines showing very poor growth were established.

When used to prepare chimeric mice, these lines resulted in chimerism as low as 50% and did not contribute to the germ line. For this reason, the GSK3 inhibitor CHIR99021 and the MEK inhibitor PD0325901, which are considered to be effective for maintenance of the undifferentiated state of ES cells, were added to the medium (GMEM-KSR-2i medium) in an attempt once again to establish ES lines.

More specifically, BRJ embryos were collected by *in vitro* fertilization. 64 embryos were cultured in KSOM medium for 4 days until they became blastocysts, and the embryos were transferred on a one-by-one basis to 48 wells (coated with gelatin alone). The medium used was KSR-GMEM-2i medium composed of G-MEM (Glasgow minimum essential medium) supplemented with 1 X MEM nonessential amino acids, 0.1 mM β-mercaptoethanol, 1 mM sodium pyruvate, 1% fetal bovine serum (FBS) (Hyclone), 14% Knockout™ SR (KSR), 1100 uints/ml leukemia inhibitory factor (LIF), 2 µM PD0325901 and 3 µM CHIR99021. The culture period was set to 14 days, during which the medium was replaced twice. After 14 days to 18 days, subculture was conducted from wells with increased ICM to 24 wells containing feeder cells. Further, subculture was conducted sequentially in 12 wells, 6 wells and 6-cm dishes, finally establishing 28 lines of ES cells having no problem in growth rate and morphology.

### (2) Preparation of chimeric mice using BRJ ES cell lines and establishment of BRJ Rag2-/-;Jak3-/- mouse strains

Among the established ES lines, 8 cell lines were used to prepare chimeric mice by being aggregated with morula embryos obtained by crossing between B6 female and BDF1 male mice (Table 1).

Germ-line transmission was confirmed in 100% chimeras obtained from three ES lines (BRJ-5, BRJ-6 and BRJ-8) (Table 2).

It should be noted that among the resulting ES cells, the 8th cell line was designated as "BRJ8" and internationally deposited under the Budapest Treaty on March 23, 2012 (receipt date) with the National Institute of Technology and Evaluation, Patent Microorganisms Depositary (Patent Microorganisms Depositary, Department of Biotechnology of NITE, 2-5-8 Kazusakamatari, Kisarazu-shi, Chiba 292-0818, Japan). Its Accession No. is "NITE BP-1297."

**Table 1**

| **Line No.** | **Transfer** | **Foster** | **Newborn** | **White eyes** | **100% chimera** |
|---|---|---|---|---|---|
| **3** | **88** | **3** | **3** | **3** | **3 ♀** |
| **4** | **60** | **2** | **0** | **0** | |
| **5** | **125** | **5** | **23** | **23** | **18 ♂** |
| **5** **4X passage** | **100** | **4** | **23** | **22** | **5 ♂** |
| **6** | **125** | **5** | **13** | **11** | **3 ♂, 4♀** |
| **7** | **75** | **3** | **0** | **0** | |
| **8** | **75** | **3** | **17** | **17** | **Died before weaning** |
| **8** **4X passage** | **125** | **5** | **15** | **13** | **7♂** |
| **9** | **75** | **3** | **16** | **16** | **12 ♀** |
| **10** | **75** | **3** | **23** | **23** | **19 ♀** |

**Table 2**

| **ES line** | **Born** | **Copy No.** | | **Chimerism (♂/♀)** | | | | | |
|---|---|---|---|---|---|---|---|---|---|
| | | **SD** | **CD** | **<10%** | **10% -20%** | **20% -40%** | **40% -60%** | **60% -80%** | **80% -100%** |
| **BRJ5-SDCD09** | **11.06.27** | **2** | **2** | **-/-** | **-/-** | **-/-** | **-/-** | **-/-** | **-/1** |
| **BRJ5-SDCD18** | **11.07.04** | **1** | **3** | **2/-** | **-/-** | **-/-** | **-/-** | **-/-** | **-/-** |
| **BRJS-SDCD41** | **11.07.04** | **3** | **5** | **5/-** | **-/-** | **1/-** | **-/-** | **-/-** | **-/ -** |
| **BRJ6-SDCD42** | **11.06.27** | **-** | **-** | **-/-** | **-/-** | **-/-** | **-/4** | **-/2** | **-/4** |
| **BRJ5-SDCD49** | **11.06.27** | **1** | **2** | **2/-** | **3/-** | **3/-** | **-/-** | **-/ -** | **-/-** |
| **BRJ5-SDCD55** | **11.07.11** | **2** | **4** | **-/-** | **5/-** | **3/-** | **2/-** | **3/-** | **2/-** |
| **BRJ5-SDCD68** | **11.06.27** | **1** | **2** | **3/-** | **-/-** | **4/-** | **-/-** | **-/-** | **-/-** |
| **BRJ8-SDCD13** | **11.08.08** | **1** | **2** | **4/-** | **1/-** | **3/-** | **1/-** | **2/-** | **-/-** |
| **BRJ8-SDCD16** | **11.08.08** | **2** | **6** | **3/-** | **-/-** | **1/-** | **3/-** | **-/-** | **-/-** |
| **BRJ8-SDCD20** | **11.08.08** | **-** | **-** | **4/-** | **1/-** | **1/-** | **-/-** | **-/-** | **-/-** |
| **BRJ8-SDCD27** | **11.08.08** | **1** | **5** | **3/-** | **1/-** | **-/-** | **-/-** | **-/-** | **-/-** |
| **BRJ8-SDCD31** | **11.08.10** | **-** | **-** | **3/-** | **1/-** | **1/-** | **-/-** | **-/-** | **-/-** |
| **BRJ8-SDCD32** | **11.08.10** | **1** | **5** | **5/-** | **-/-** | **-/-** | **-/-** | **-/-** | **-/-** |

### [Example 2] Induction of cell death in mouse liver cells

### (1) Preparation of constructs for induction of cell death in mouse liver cells

For preparation of a genetically modified mouse capable of specifically causing death in liver cells, two constructs were prepared.

Construct 1 (CAG-ATG-rox-EGFP-rox-DT-A) is composed of ATG, EGFP flanked by rox sites and DT-A (diphtheria toxin fragment A), which are ligated immediately downstream of the CAG promoter.

This construct was designed to ensure in-frame ligation between the initiation codon in EGFP and ATG located upstream of rox. This construct was also designed to remove the initiation codon in DT-A and to ensure in-frame ligation with ATG located upstream of rox.

Construct 2 (SAP-DreER^{T2}) is composed of Dre-ER^{T2} ligated immediately downstream of the promoter for liver cell-specific serum amyloid P component (SAP). In addition, the puromycin resistance gene is ligated upstream of the SAP promoter.

Detailed procedures are as shown below.

### (1-1) Construct 1

Construct 1 was prepared in the following manner.
(i) p6SEAZ and pSP-rox2 were treated with restriction enzymes PstI and KpnI, respectively, and then blunt-ended with T4 Polymerase (TaKaRa). Subsequently, they were treated with a restriction enzyme EcoRI and ligated to each other to prepare pSP-rox-EGFP-rox.
(ii) pSP-rox-EGFP-rox and pBSK-atg-rox2 (synthetic DNA, Biomatik) were treated with restriction enzymes EcoRI and SmaI, and then ligated to each other to prepare pBSK-atg-rox-EGFP-rox.
(iii) pBSK-atg-rox-EGFP-rox and P71hAXC-DT were treated with restriction enzymes BamHI and PstI, and then ligated to each other to prepare pBSK-atg-rox-EGFP-rox-DT-A.
(iv) pCAGGS-EGFP and pBSK-atg-rox-EGFP-rox-DT-A were treated with restriction enzymes KpnI and SpeI, respectively, and then blunt-ended with T4 Polymerase (TaKaRa). Subsequently, they were treated with a restriction enzyme Hind III and then ligated to each other to prepare CAG-atg-rox-EGFP-rox-DT-A.

### (1-2) Construct 2

Construct 2 was prepared in the following manner.
(i) pkSAP-DrePP was used as a template in PCR to amplify a region covering from the initiation codon to the last codon before the stop codon. The reverse primer was provided with a BamHI site.
   PCR kit: TaKaRa Ex Taq
   Fw Primer: CCATGGCCCCCAAGAAGAAAA (SEQ ID NO: 14)
   Re Primer: CGGGATCCATGAGCCTGCTGTT (SEQ ID NO: 15)
   pGEM-T Easy Vector and the above PCR product were ligated to each other to prepare T easy-Dre.
(ii) pkSAP-DrePP and T easy-Dre were treated with restriction enzymes SalI and EcoRI, and then ligated to each other to prepare T Easy SAP.
(iii) The above T Easy Dre and T easy-SAP were treated with restriction enzymes SacII and NotI, and then ligated to each other to prepare T easy-SAP-Dre.
(iv) T Easy-SAP-Dre and pkSA-CremER^{T2}PP were used and treated with restriction enzymes BamHI and NotI, and then ligated to each other to prepare T easy-SAP-DremER^{T2}.
(v) pkSAP-DrePP and T easy-SAP-DremER^{T2} were treated with restriction enzymes SalI and NotI, and then ligated to each other to prepare pKSAP-DreER^{T2}.
(vi) pKSAP-DreERT2 and pFPacpaF2 were treated with restriction enzymes SpeI and KpnI, respectively, and then blunt-ended with T4 polymerase (TaKaRa). Subsequently, pKSAP-DreER^{T2} and pFPacpaF2 were treated with restriction enzymes SalI and XhoI, respectively, and then ligated to each other to prepare Puro-SAP-DreER^{T2}.

### (2) Introduction of estrogen receptor gene and diphtheria toxin gene into ES cells

Conditions were studied to ensure efficient expression of human genes upon insertion (Li, Z. et al., Transgenic Res. 20:191-200, 2011. DOI 10.1007/s11248-010-9389-22).

The presence or absence of a PGK-puromycin cassette and IRES was analyzed to determine which combination would achieve the highest expression efficiency.

Prior to the analysis, a homologous recombination vector was used to disrupt the first exon of the mouse transthyretin (Ttr) gene in a standard manner (Zhao, G., Li, Z., Araki, K., Haruna, K., Yamaguchi, K., Araki, M., Takeya, M., Ando, Y. and Yamamura, K. Inconsistency between hepatic expression and serum concentration of transthyretin in mice humanized at the transthyretin locus. Genes Cells 13: 1257-1268, 2008). During this treatment, ATG in the first exon was disrupted, resulting in a target recombinant clone carrying lox71-PGK-beta-geo-loxP-polyA-lox2272 integrated into this site.

Then, two types of replacement vectors were prepared. Replacement vector 1 comprises lox66-hTTR cDNA-polyA-Frt-PGK-puro-Frt-loxP, while replacement vector 2 comprises lox66-IRES-hTTR cDNA-polyA-Frt-PGK-puro-Frt-loxP. These replacement vectors were each introduced together with a Cre expression vector into the target recombinant clone by electroporation.

As a result, the following two clones were obtained: lox71/66-hTTR cDNA-polyA-Frt-PGK-puro-Frt-loxP (abbreviated as I(-)P(+)) and lox71/66-IRES-hTTR cDNA-polyA-Frt-PGK-puro-Frt-loxP (abbreviated as I(+)P(+)). These two clones each have PGK-puro, but I(-)P(+) has no IRES.

Into these two clones, CAG-FLP was introduced by electroporation and PGK-puro between Frt sites was deleted to prepare I(-)P(-) and I(+)P(-) clones.

Mice were prepared from these four ES clones and subjected to expression analysis, indicating that I(-)P(+) showed the highest expression, followed by I(-)P(-), I(+)P(+) and I(+)P(-) in decreasing order. Moreover, in the case of I(-)P(+), human TTR (transthyretin) expression in the liver was found to be substantially equal to the expression levels of mouse Ttr (transthyretin) in control mice.

As a result, a combination of the presence of PGK-puromycin and the absence of IRES was found to achieve the highest expression efficiency for the inserted human gene.

### [Example 3] Replacement with human growth hormone gene

Prior to the experiment, a homologous recombination vector was used to disrupt the first exon of the mouse growth hormone (Gh) gene in a standard manner as in the case of Example 2. During this treatment, ATG in the first exon was disrupted, resulting in a target recombinant clone carrying lox71-PGK-beta-geo-loxP-polyA-lox2272 integrated into this site. Then, a replacement vector was prepared. The replacement vector comprises lox66-hGH cDNA-polyA-Frt-PGK-puro-Frt-loxP. This replacement vector was introduced together with a Cre expression vector into the target recombinant clone by electroporation.

As a result, an ES clone in which the mouse Gh gene was replaced with the human GH gene was obtained.

### [Example 4] Replacement with human drug-metabolizing enzyme gene

Prior to the experiment, a homologous recombination vector was used to disrupt the first exon of the mouse Cyp3a13 gene in a standard manner. During this treatment, ATG in the first exon was disrupted, resulting in a target recombinant clone carrying lox71-PGK-beta-geo-loxP-polyA-lox2272 integrated into this site. Then, a replacement vector was prepared. The replacement vector comprises lox66-hCYP3A4 cDNA-polyA-Frt-PGK-puro-Frt-loxP. This replacement vector was introduced together with a Cre expression vector into the target recombinant clone by electroporation.

As a result, an ES clone in which the mouse Cyp3a13 gene was replaced with the human CYP3A4 gene was obtained.

### [Example 5] Preparation of mouse whose liver is humanized

Techniques to induce differentiation from human iPS cells into human liver cells were substantially established, and constructs for induction of cell death in mouse liver cells were also prepared.

### (1) Induction of differentiation from human iPS cells into liver cells

Efficient techniques were constructed for induction of endodermal and hepatic differentiation from human iPS cells with the use of supporting cells or an extracellular matrix.

For purification of differentiated liver cells and for evaluation of differentiation induction efficiency, a human iPS cell line carrying an albumin-reporter gene was established and used for development of differentiation induction techniques. Culture matrixes used for this purpose include M15 cells serving as supporting cells (feeder cells), a synthesized basement membrane (sBM), Cell-able and so on.

To cause differentiation from iPS cells into human liver cells, a 4,500 mg/l glucose DMEM medium was used for culture from the first day to the 9th day. This DMEM medium contains the following: insulin (10 mg/l), transferrin (5.5 mg/l), sodium selenite (6.7 mg/ml), ALBUMAX II (2.5 mg/ml), 100 mM nonessential amino acids, 2 mM L-glutamine, 50 mg/ml streptomycin, 100 µM β-mercaptoethanol, activin A (20 ng/ml), and bFGF (50 ng/ml).

Subsequently, culture was continued in the presence of retinoic acid (10⁻⁶ M) from the 9th day to the 11th day.

Finally, from the 11th day to the 30th day, the cells were transferred to and cultured in a 2,000 mg/l glucose DMEM medium to complete their differentiation into liver cells. The DMEM medium used for this purpose contains the following: 10% KSR, 100 mM nonessential amino acids, 2 mM L-glutamine, 50 mg/ml streptomycin, 100 µM β-mercaptoethanol, hepatocyte growth factor (10 ng/ml), dexamethasone (1 mM), dimethylsulfoxide (1%), and nicotinamide (1 mM).

### (2) Study of transplantation techniques for iPS-derived human liver cells

With the aim of establishing techniques for efficient introduction of iPS-derived liver cells into mouse livers, which are required for humanization of livers, a preliminary experiment was conducted to introduce iPS-derived human liver cells through the blood vessel (yolk sac vessel) present on the mouse fetal amniotic membrane at 17 days of embryonic age (Figures 6 and 7).

Glycerol micelles encapsulating (A) a dye (blue dye) and (B) a GFP expression vector were introduced through the blood vessel present on the amniotic membrane at 17 days of embryonic age and confirmed for their introduction efficiency.

As a result, the injectants were confirmed to be concentrated and localized in the liver and found to be throughout the liver. Moreover, the survival and delivery of fetuses were not affected even after this treatment, and neonatal mice whose livers were efficiently introduced with both injectants (A) and (B) were born.

The liver cells prepared in (1) above were used for transplantation.

Figure 8 shows liver cells induced to differentiate on M15 and sBM.

Then, to induce a large number of liver cells, culture plates under the name Cell-able (Toyo Gosei Co., Ltd., Tokyo) were used for culture.

As a result, culture for 30 days was sufficient to induce differentiation into a large number of liver cells, as expected, and spheroid formation was also observed (Figure 9).

In either culture method, iPS cells were induced to differentiate into Sox17-positive endoderm at the 10th day of culture, AFP-positive immature liver cells at the 20th day of culture, and ALBUMIN-positive mature liver cells at the 30th day of culture.

For functional evaluation of these mature liver cells induced to differentiate, glycogen accumulation was evaluated by PAS staining, while the detoxication ability was evaluated by indocyanine green (ICG) staining.

As a result, the liver cells induced to differentiate were found to have desired functions.

In addition, the liver cells showed no mouse gene expression when analyzed by RT-PCR with mouse specific primers, thus indicating that 100% of the liver cells were of human origin.

### [Example 6] Establishment of mutated humanized liver mice

In this example, FAP and PA model mice were bred.

### (1) Induction of mutated liver cells from human patients

### (i) Familial amyloid polyneuropathy (FAP): already established

FAP is an autosomal dominant hereditary disease caused by a point mutation in the transthyretin (TTR) gene. For example, in FAP, a replacement of valine with methionine occurs at amino acid position 30 in the amino acid sequence of transthyretin (Val30Met). Fibroblasts taken from patients having this Val30Met mutation were used to establish iPS cells.

As a result, it was indicated that these iPS cells were able to be induced to differentiate into liver cells in the same manner as described previously.

### (ii) Establishment of iPS cells from human propionic acidemia (PA) patients

PA is an autosomal recessive hereditary disease caused by a defect in the propionyl CoA carboxylase (PCCA) gene. For example, in PA, a replacement of arginine with tryptophan occurs at position 52 in the amino acid sequence of PCCA (Arg52Trp). Fibroblasts taken from patients having this mutation were used to establish iPS cells. As a result, it was indicated that these iPS cells were able to be induced to differentiate into liver cells in the same manner as described previously.

### (2) Establishment of mutated humanized liver mice (model mice for FAP and PA)

Mutated humanized liver mice may be established in the same manner as used to prepare a humanized liver mouse (i.e., a mouse prepared by transplantation of liver cells induced from normal human-derived iPS cells). Namely, the mouse of the present invention may be transplanted with liver cells induced to differentiate from iPS cells derived from FAP and PA patients to thereby establish the mutated humanized liver mice.

### INDUSTRIAL APPLICABILITY

The present invention provides ES cells derived from an immunodeficient mouse. An embryo prepared using the ES cells of the present invention may be transplanted with human liver cells to thereby create a mouse with a humanized liver, which can be used to examine human liver functions.

### Deposition Number

Microorganism is labeled as: "BRJ8"
Accession No.: NITE BP-1297
Initial deposit date (receipt date): March 23, 2012
International Deposition Authority:
   National Institute of Technology and Evaluation, Patent Microorganisms Depositary
   Patent Microorganisms Depositary, Department of Biotechnology of NITE, 2-5-8 Kazusakamatari, Kisarazu-shi, Chiba 292-0818, Japan

### Sequence Listing Free Text

SEQ ID NOs: 1 to 15: synthetic DNAs

## Claims

1. An embryonic stem cell obtainable from an embryo of an immunodeficient mouse which is deficient in both Rag2 and Jak3 genes by culture in the presence of a GSK3 inhibitor and an MEK inhibitor.

2. The embryonic stem cell according to claim 1, which is deposited under Accession No. NITE BP-1297.

3. The embryonic stem cell according to claim 1 or 2, which is engineered to have the estrogen receptor gene and the diphtheria toxin gene.

4. The embryonic stem cell according to claim 3, wherein the endogenous growth hormone gene in the cell is replaced with that of human origin.

5. The embryonic stem cell according to claim 4, wherein an endogenous drug-metabolizing enzyme gene in the cell is further replaced with that of human origin.

6. The embryonic stem cell according to claim 5, wherein the endogenous drug-metabolizing enzyme gene in the cell is at least one selected from the group consisting of Cyp3a11, Cyp3a13, Cyp3a25 and Cyp3a41.

7. A mouse, which is created with the use of the embryonic stem cell according to claim 1 or 2.

8. A transgenic mouse, which is created with the use of the embryonic stem cell according to any one of claims 3 to 6.

9. The mouse according to claim 8, which develops liver cell injury upon administration of an antiestrogen.

10. A mouse with a humanized liver, wherein the mouse according to claim 8 is transplanted with liver cells of human origin and also administered with an antiestrogen to eliminate liver cells originating from the mouse.

11. The mouse according to claim 10, wherein the liver cells of human origin are derived from a patient with a liver disease.

12. A human liver disease model mouse, which consists of the mouse according to claim 11.

13. A method for preparing an immunodeficient mouse-derived embryonic stem cell, which comprises culturing an embryo of an immunodeficient mouse which is deficient in both Rag2 and Jak3 genes in the presence of a GSK3 inhibitor and an MEK inhibitor.

14. A method for creating a liver injury model mouse, which comprises administering an antiestrogen to the mouse according to claim 8.

15. A method for creating a mouse with a humanized liver, which comprises transplanting liver cells of human origin into the mouse according to claim 8 and also administering an antiestrogen to eliminate liver cells originating from the mouse.

16. The method according to claim 15, wherein the liver cells of human origin are derived from a patient with a liver disease.


SEQUENCE LISTING

<110>  Trans Genic Inc.
       Kumamoto University

<120>  Humanized mouse

<130>  PCT12-0010

<160>  15

<170>  PatentIn version 3.3

<210>  1
<211>  34
<212>  DNA
<213>  Artificial

<220>
<223>  Synthetic DNA

<400>  1
ataacttcgt atagcataca ttatacgaag ttat                                34

<210>  2
<211>  34
<212>  DNA
<213>  Artificial

<220>
<223>  Synthetic DNA

<400>  2
taccgttcgt atagcataca ttatacgaag ttat                                34

<210>  3
<211>  34
<212>  DNA
<213>  Artificial

<220>
<223>  Synthetic DNA

<400>  3
ataacttcgt atagcataca ttatacgaac ggta                                34

<210>  4
<211>  34
<212>  DNA
<213>  Artificial

<220>
<223>  Synthetic DNA

<400>  4

ataacttcgt atagtataca ttatacgaag ttat 34

<210> 5
<211> 34
<212> DNA
<213> Artificial

<220>
<223> Synthetic DNA

<400> 5
ataacttcgt ataggatact ttatacgaag ttat 34

<210> 6
<211> 34
<212> DNA
<213> Artificial

<220>
<223> Synthetic DNA

<400> 6
tcaatttctg agaactgtca ttctcggaaa ttga 34

<210> 7
<211> 34

<212>  DNA
<213>  Artificial

<220>
<223>  Synthetic DNA

<400>  7
cgtgattctg agaactgtca ttctcggaaa ttga                                    34

<210>  8
<211>  34
<212>  DNA
<213>  Artificial

<220>
<223>  Synthetic DNA

<400>  8
tcaatttctg agaactgtca ttctcggaat acct                                    34

<210>  9
<211>  34
<212>  DNA
<213>  Artificial

<220>
<223>  Synthetic DNA

<400> 9
tcaatttctg agaagtgtct ttctcggaaa ttga 34

<210> 10
<211> 32
<212> DNA
<213> Artificial

<220>
<223> Synthetic DNA

<400> 10
taactttaaa taatgccaat tatttaaagt ta 32

<210> 11
<211> 32
<212> DNA
<213> Artificial

<220>
<223> Synthetic DNA

<400> 11
taactttaaa taattggcat tatttaaagt ta 32

<210> 12
<211> 34
<212> DNA
<213> Artificial

<220>
<223> Synthetic DNA

<400> 12
ataacttcgt ataatgtatg ctatacgaag ttat 34

<210> 13
<211> 34
<212> DNA
<213> Artificial

<220>
<223> Synthetic DNA

<400> 13
ataacttcgt atagcataca ttatacgaag ttat 34

<210> 14
<211> 21
<212> DNA
<213> Artificial

<220>
<223>  Synthetic DNA

<400>  14
ccatggcccc caagaagaaa a                                                21

<210>  15
<211>  22
<212>  DNA
<213>  Artificial

<220>
<223>  Synthetic DNA

<400>  15
cgggatccat gagcctgctg tt                                               22